# EUROPEAN PATENT APPLICATION

(11) **EP 4 289 817 A1**
(43) Date of publication of application: **13.12.2023**
(21) Application number: 22177609.9
(22) Date of filing: 07.06.2022
(51) Int. Cl.: C07C 273/04, C07C 273/16

(54) **PROCESS FOR REMOVING BIURET FROM UREA**

(71) Applicant: CASALE SA, 6900 Lugano (CH)
(72) Inventor: MARRONE, Leonardo, 21020 Mercallo (VA) (IT); BENEDETTI, Alberto, 22100 Como (IT)
(74) Representative: M. Zardi & Co S.A.

(57) **Abstract**

A process for removing biuret for solid urea (UR), wherein the solid urea is in a granular or powder form, the process including leaching the solid urea with a leaching solution (LS) which is a urea saturated aqueous solution, wherein biuret contained in the solid urea is transferred to said leaching solution during the leaching process, obtaining a purified solid urea.

## Description

### Field of the invention

The invention relates to the field of production of urea.

### Prior Art

Urea is produced industrially by reacting ammonia and carbon dioxide at suitable urea-forming conditions, typically at a high pressure and high temperature.

Urea is synthesized at a synthesis pressure above 100 bar obtaining a reaction effluent containing urea, water and unconverted reagents mostly in the form of ammonium carbamate. Said reaction effluent is normally processed in one or more recovery sections at a pressure lower than the synthesis pressure, obtaining a recycle solution containing the reagents removed from the effluent, and a purified aqueous solution of urea. Said purified solution typically contains around 65-70% urea, the balance being water and unavoidable impurities.

The process of recovery normally includes heating the solution to decompose the ammonium carbamate into ammonia and carbon dioxide; a gaseous phase containing ammonia and carbon dioxide is then removed and condensed to obtain a recycle solution which can be sent back to the reactor.

In the widely used stripping processes, the effluent of a high-pressure reactor is heated in a high-pressure stripper, possibly in the presence of a stripping agent, to decompose the ammonium carbamate and extract gaseous ammonia and carbon dioxide. These are condensed in a high-pressure condenser and recycled to the synthesis reactor. When used, the stripping agent is generally gaseous carbon dioxide or gaseous ammonia.

Said high-pressure stripper and high-pressure condenser may operate at substantially the same pressure as the synthesis reactor, thus forming a high-pressure synthesis section or loop. The urea-containing effluent of the stripper is then processed in one or more recovery sections as described above.

The impurities found in the purified solution include biuret. Urea is subject to thermal decomposition into biuret and ammonia and therefore some biuret may inevitably form in the process. In the conventional production of urea, biuret is an undesired by-product and efforts are made to avoid its formation. Certain applications of urea require a stringent limit on the maximum content of biuret, for example not greater than 1.5 or 1.0% by weight, or even less. Applications with such stringent limit include, for example, fertilizer foliar-grade urea, technical-grade urea, urea for the production of diesel exhaust fluid (DEF). Quality requirements for DEF can be found in the DIN 70070 Norm. On the other hand, the biuret itself has some interesting applications, for example urea with a high content of biuret can be used for cattle feeding.

In most cases, the urea solution withdrawn from the recovery section is converted in a solid form. The techniques for converting the solution into a solid product include a concentration step by crystallization or evaporation and a subsequent step of product shaping by granulation or prilling. Granulation requires a highly concentrated solution (at least 95-96% of urea) whereas prilling requires an almost pure urea melt (>99.5% urea). All the above processes are well known to a skilled person.

The crystallization process includes that the urea solution is heated and depressurized to vacuum, typically around 0.1 bar abs, so that water evaporates from the solution; then urea crystals are produced by crystallization and separated from the aqueous phase (mother liquor) by suitable means, such as a combination of sieve bends and centrifuges. The crystals are then melted to produce a urea melt; the urea melt feeds a prilling tower or granulation unit.

In the evaporation process, the solution is heating under vacuum to remove water, obtaining directly (without solidification and subsequent melting of solid crystals) the concentrated solution or urea melt suitable for granulation or prilling. In this process step some biuret is unavoidably formed because of the high temperature close to urea melting point and vacuum conditions.

The crystallization process can produce urea with a very low content of biuret. If kept below the saturation point in the urea solution, the biuret remains in the liquid and does not crystallize together with urea. Crystals of urea with less than 0.9 %wt (% by weight) can be obtained, which are subsequently melted to produce a liquid urea melt to feed a prilling tower or granulator. In contrast, the concentration of the urea solution by simple evaporation is not always able to give such low-biuret urea, but is more common in modern urea plants and is generally preferred because it does not need installation of a urea melter and related means for handling solid crystals of urea.

In summary, the request for low-biuret urea is growing. However, the majority of urea plants have an evaporation-based concentration section which may not be able to reach the required purity. Installation of a dedicated crystallization section is very expensive and therefore not attractive.

### Summary of the invention

The invention aims to improve the above discussed prior art. A first aim of the invention is to provide a new process for production of urea with a low content of biuret, such as less than 1.0% or less than 0.9% by weight. Another aim of the invention is to provide a process suitable for production of low-biuret urea, starting from the urea solution after recovery, which can be implemented with concentration by evaporation followed by granulation or prilling, obtaining solid urea with a purity, in terms of low content of biuret, comparable to that obtainable with concentration performed by crystallization and subsequent melting of the crystals of urea. Another aim of the invention is to provide a process which can produce low-biuret urea together with high-biuret urea, whenever the latter is also of industrial interest.

The aims are reached with a process according to claim 1. The invention provides that biuret is removed from solid urea with a leaching process. The leaching is performed with an aqueous solution of urea as leaching solution. In the leaching process, biuret contained in the solid urea is transferred to the leaching solution, thus obtaining a purified solid urea and a leaching solution enriched with biuret.

In some embodiments, a portion of said solution, after the leaching process, may be processed for obtaining biuret or high-biuret urea, which may be an additional valuable output of the process.

In the invention, the biuret is removed directly from solid urea, which may be in a granular or powder form. The invention does not require expensive items and can be implemented in urea plant with evaporation-based concentration section, obtaining urea with a low biuret content comparable to that obtainable from a crystallization-based concentration section.

### Description of the invention

The invention concerns a process for removing biuret for solid urea, wherein the solid urea is in a granular or powder form, the process including leaching the solid urea with a leaching solution which is an aqueous solution of urea and is saturated with urea, wherein biuret contained in the solid urea is transferred to the solution, obtaining a purified solid urea, which is then separated from the leaching solution.

The urea in granular form may be urea obtained from a prilling process or from a granulation process. In an embodiment the solid urea is in the form of spherical granules having an average diameter not greater than 3.5 mm, for example in the range 2.0 mm to 3.5 mm. In some embodiments the granular urea may be urea obtained from a pelletizer, e.g. from a rotoformer.

The leaching process is preferably performed at a controlled temperature. In preferred embodiments the temperature of the leaching process is not greater than 80 °C, more preferably in the range 20 °C to 80 °C, even more preferably in the range 50 °C to 70 °C, or 55 °C to 65 °C, such as 60 °C or about 60 °C.

Very preferably, the leaching process is performed at a substantially constant temperature. Both the solid urea and the leaching solution are preferably fed at the same temperature at which the process is performed, which may be termed leaching temperature. In a preferred embodiment both the solid urea and the leaching solution are at 60 °C or about 60 °C.

The appropriate contact time between the solid urea and the leaching solution depends mainly on the size distribution of the urea granules, fluid dynamic field and the leaching temperature. In preferred embodiments contact between the solid urea and the leaching solution is provided under intensive mechanical agitation (e.g. about 10 hp/1000 gal) to enhance the mass transfer of biuret. In preferred embodiments of the invention, the contact time is 15 minutes to 120 minutes. For a given temperature, it has been noted that a fine size of the granules renders the leaching process more efficient and, consequently, a short contact time within the above range may be sufficient. Particularly, a contact time of 15 min to 45 min is preferred for urea in a powder form, whereas a contact time of 60 min to 120 min is preferred for urea in a granular form.

The leaching process can be performed in a suitable equipment, also termed leacher, fed with the solid urea and the leaching solution. Said leacher is preferably a stirred tank reactor. The above-mentioned contact time may be, accordingly, a residence time of the solid urea and leaching solution in the leacher.

In the leaching process of the invention, the weight ratio between the solid urea to be processed and the leaching solution is preferably between 1.0 and 4.0, more preferably between 1.5 and 2.5.

After the leaching process, for example after the selected residence time in the leacher, the purified solid urea is separated from the leaching solution in a suitable apparatus, preferably by centrifugation and/or by filtering. More preferably, the separation process is performed at the same temperature or substantially the same temperature as the leaching process to avoid precipitation of additional solid phase.

The leaching solution separated from the purified solid urea can be partly recycled to the leaching process, e.g. reintroduced into the leacher, if the biuret contained in the solution so permits.

In an embodiment, the leaching solution separated from the purified urea is split into a first portion which is directly recycled to the leaching process, forming a main recycle stream, and a purge solution. Said purge solution is a minor portion of the leaching solution separated from the purified urea, for example 1% to 20% or preferably 2% to 10%, generally around 5%.

Said main recycle solution forms a main portion of the leaching solution and has a significant content of biuret (previously removed from the solid urea). Therefore, it may be necessary to implement steps to control the amount of biuret in the leaching solution.

For practical reasons, it may be preferred to control the concentration of biuret in the effluent stream of the leaching process. For example, the leaching process is performed in a leaching apparatus; the separation of the solution from the purified urea is performed in a separator downstream the leacher; the amount of said purge solution is controlled to maintain a target concentration of biuret, below saturation, in the leaching solution at the outlet of said leaching apparatus.

The biuret content in the leaching solution effluent from the leaching process may be preferably controlled to remain in the range 15 wt% to 18 wt%. Said target concentration of 15 to 18% biuret is particularly preferred in combination with a temperature of the leaching process in the range 50 °C to 70 °C, more preferably at or around 60 °C.

According to preferred embodiments, the concentration of biuret in the leaching solution may be controlled:
by processing the purge solution to remove biuret contained therein, and joining a so obtained biuret-depleted secondary recycle stream of urea solution with said main recycle stream, and/or
by recycling the purge solution to the urea plant so that it is forced passing through the urea reactor and adding the main recycle stream with a make-up of fresh aqueous urea solution, said make-up solution being saturated in urea and containing no biuret or having a content of biuret lower than that of said main recycle stream.

In the first option, the processing of the purge solution can be carried out in a dedicated section. In the second option, the purge solution is preferably recycled to a tied-in urea plant. The recycling of the purge solution to said urea plant includes preferably that the purge solution is passed through a urea synthesis reactor.

The first option is of particular interest when a market exists also for biuret, for example as a cattle feed. In that case, the biuret removed from the purge solution may represents an added value. The processing of the purge solution therefore may have the combined advantages of controlling the biuret in the leaching solution and obtaining biuret as a further valuable product. The so obtained biuret may be substantially pure biuret or may contain some urea and be termed a high-biuret urea. In this option the biuret concentration in the purge stream is preferably controlled higher than 10%wt, more preferably higher that 15%wt to increase the productivity of biuret.

The second option may include that solid urea is dissolved with water to obtain said make-up solution. Said solid urea may be a portion of the purified urea obtained after the leaching process. Alternatively, said make-up solution may be a saturated urea aqueous solution obtained by diluting a highly concentrate urea melt with water.

The option of processing the purge solution to remove biuret may include a biuret crystallization step. The purge solution, which is relatively hot and contains little water, may be added with water and cooled prior to said crystallization step, to reach conditions suitable for the crystallization of biuret. In preferred embodiments the solution sent to crystallization of biuret contains 40% to 60% water, preferably 45% to 55%, and has a temperature of 10 °C to 30 °C, preferably 10 °C to 20 °C. The crystallization of biuret is performed preferably at about 5 °C.

A slurry obtained in the biuret crystallization process can be separated into a solid phase of biuret-containing crystals and a biuret crystallization liquor; said liquor can be subsequently concentrated by evaporation to remove water and to obtain a secondary recycle stream for the leaching process. Said secondary stream may be joined to the above mentioned main recycle stream taken directly from the liquid-solid separation performed after leaching.

The crystallization of biuret removes heat, so that the slurry is colder than the feed solution. In a preferred embodiment the feed solution is at about 15 °C and the slurry is at about 5 °C.

Said evaporation of the biuret crystallization liquor is preferably regulated to obtain a liquid solution saturated with urea. The water removed from the liquor during said evaporation step may be condensed and used to dilute the purge solution before the crystallization of biuret. Therefore, at least part of an aqueous stream added to the purge solution may be obtained from water removed from said liquor.

Said evaporation step may be regulated to obtain a liquid solution saturated with urea at the temperature of the leaching process. Preferably, said evaporation step is performed under vacuum, more preferably at a pressure of 0.08 to 0.15 bar abs, such as 0.10 bar abs.

In a preferred embodiment the purge solution, before the crystallization of biuret, is cooled by transferring heat to said crystallization liquor, so that the liquor is also pre-heated before the evaporation step. This can be made in an indirect heat exchanger, such as a tube heat exchanger or a plate heat exchanger, wherein a one side is traversed by the hot purge solution and another side is traversed by the liquor.

For example the purge solution may be cooled from 60 °C to around 15 °C, whereas the liquor may be heated from around 5 °C to 50 °C.

The processing of the purge solution, with particular reference to the crystallization of biuret, may be performed in accordance with various embodiments described in WO 2022/106083.

Certain embodiments may include a launch phase during which a leaching urea solution, initially containing no biuret, is continuously recycled to the leaching process without removing a purge stream until a target concentration of biuret is reached in the solution effluent from the leaching process. At the end of this launch phase, that is when the target concentration has been reached, the process may continue as above described, by removing the purge stream to maintain a desired concentration of biuret in the leaching solution.

In some embodiments the process can be performed discontinuously, e.g. the leaching solution, once a target concentration of biuret is reached due to recycle, may be processed separately to remove the biuret contained therein, so that a substantially biuret-free solution is recovered.

The invention can be applied to a urea production process wherein an aqueous solution of urea, obtained from a recovery section, is concentrated in an evaporation section and the so obtained concentrated solution or urea melt feeds a prilling process or a granulation process. The so obtained prills or granules can be processed, in accordance with the present invention, to remove biuret contained therein.

An embodiment of the invention is a process for producing low-biuret urea comprising:
a urea aqueous solution is produced by reacting ammonia and carbon dioxide under urea-forming conditions, preferably according to a urea stripping process;
said solution is subject to evaporation to remove water and obtain a concentrated urea solution or a urea melt;
said concentrated urea solution or urea melt is processed by prilling or granulation to produce solid urea;
at least a portion of said solid urea is processed to remove biuret with a leaching process as above described.

The production of the urea aqueous solution may include the production of a urea-containing solution in a high-pressure synthesis section and subsequent processing of said solution in one or more recovery sections, such as a low-pressure recovery section or a medium-pressure recovery section followed by a low-pressure recovery section.

The invention can also be implemented as a revamping of an existing urea plant by installation of a suitable apparatus adapted for the leaching process as above described.

Particularly, an aspect of the invention is a method for revamping a urea plant comprising a synthesis section and a recovery section arranged to produce an aqueous solution of urea, an evaporation section arranged to remove water from said solution to produce a concentrated solution or urea melt, a shaping section wherein said concentrated solution or urea melt is converted into solid urea, the method being characterized by the installation of a leaching apparatus arranged to process at least a portion of said solid urea to remove biuret with a leaching process, said apparatus being configured to leach the solid urea with an aqueous solution of urea used as a leaching agent.

A method of revamping according to embodiments of the invention may also include the installation of a biuret crystallization section configured to remove biuret from a purge stream removed from the leaching solution, as above described.

### Description of the figures

The invention and its advantages are now elucidated with the help of the figures wherein:
Fig. 1 illustrates an embodiment of the invention for production of a diesel exhaust fluid solution.
Fig. 2 illustrates an embodiment of the invention similar to Fig. 1 for production of low-biuret urea.
Fig. 3 illustrates another embodiment of the invention.

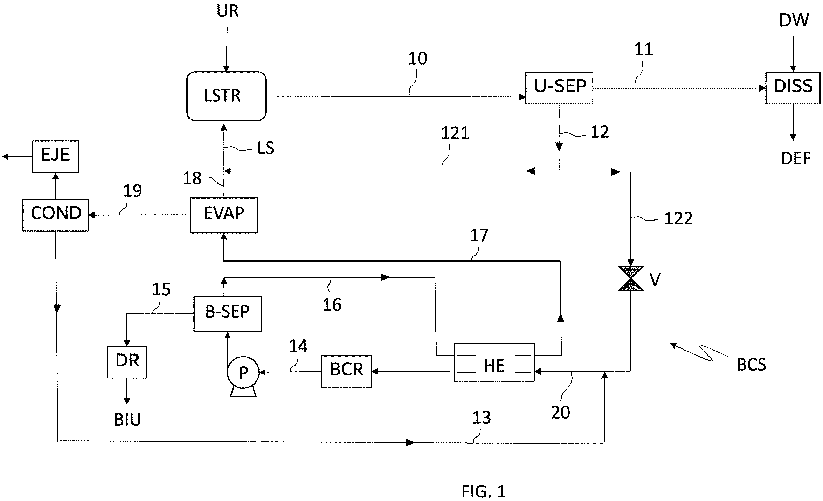

Fig. 1 illustrates the following main items.
LSTR Leaching stirred reactor.
U-SEP Liquid-solid separator for separation of the purified solid urea from the leaching solution, contained in the effluent of the reactor LSTR.
DISS Dissolver wherein the low-biuret solid urea, obtained in the reactor LSTR and isolated from the solution in the separator U-SEP, is dissolved with demineralized water DW to obtain a diesel exhaust fluid solution DEF. Such solution DEF typically contains 32.5 wt% urea.
BCS Biuret crystallization section
V Valve arranged to control the amount of leaching solution processed in the section BCS.
HE Heat exchanger.
BCR Biuret crystallizer.
B-SEP Liquid-solid separator for separation of solid biuret obtained in the crystallizer BCR.
DR dryer for removing residual water from the solid biuret, obtaining a biuret product BIU.
P Slurry pump arranged to feed the slurry effluent of the crystallizer BCR to the separator B-SEP.
EVAP Evaporator to remove water from the liquid fraction withdrawn from the separator B-SEP, before it is recycled to the leaching reactor LSTR.
COND condenser.
EJE Air ejector to maintain the condenser COND under vacuum.

Solid urea UR, for example in the form of granules or powder, is leached in the reactor LSTR with a leaching solution LS which is a saturated aqueous solution of urea.

The effluent 10 of the reactor LSTR contains purified urea and the leaching solution loaded with the biuret removed from the urea. Said effluent 10 is separated in the separator U-SEP into solid urea directed to the dissolver DISS via line 11, and a liquid fraction 12.

The liquid fraction 12 is split into a major portion 121 which is directly recycled to the reactor LSTR and a minor portion 122, also termed purge solution, which is processed in the biuret crystallization section BCS.

In said biuret crystallization section BCS, biuret is removed from the purge solution 122 to produce a biuret product BIU and to control the amount of biuret in the solution LS. The flow rate of purge solution 122 is controlled with the valve V to maintain a target concentration of biuret in the solution LS and, consequently, in the effluent 10 of the reactor LSTR. Particularly preferably, the amount of purge solution 122 is controlled as a function of the concentration of biuret in the effluent 10, to maintain said concentration below saturation level. The content of biuret in the stream 10 may be detected and the valve V controlled in accordance to increase or decrease the purge solution 122. Typically, the purge solution 122 may be 2% to 10% of the entire solution 12.

The solution 122 is cooled in the heat exchanger HE and added with recycle water 13 to achieve conditions suitable for crystallization of biuret in the crystallizer BCR. The effluent 14 of said crystallizer BCR is fed to the separator B-SEP; the solid fraction 15 is dried in the dryer DR to form the biuret product BIU; the liquid fraction 16, which is a biuret-depleted urea solution, is preheated in the heat exchanger HE with heat removed from the solution 122, and pre-heated solution 17 is sent to the evaporator EVAP.

The effluent 14 is a slurry containing a liquid solution and biuret crystals, which is sent to the separator B-SEP via the slurry pump P. The biuret product BIU is biuret at high purity, typically greater than 70%, the balance being urea, residual water and impurities.

In the evaporator EVAP, water is removed from the preheated solution 17 to achieve a concentration of urea suitable for the leaching process. The concentrated solution 18 withdrawn from the evaporator EVAP is joined with the solution 121 coming from the separator U-SEP, to form the feed of leaching solution LS.

The solution 18 forms a secondary recycle stream which is joined with the main recycle stream 121. As the solution 18 contains no or little biuret, compared to the relatively biuret-rich solution 121, it allows to adjust the content of biuret in the stream LS and, consequently, in the effluent 10.

The vapor stream 19 removed from the evaporator EVAP is condensed in the vacuum condenser COND to form the water-rich stream 13 which, as mentioned above, dilutes the purge solution 122 upstream the crystallizer BCR, preferably upstream the heat exchanger HE as illustrated.

The heat exchanger HE is an indirect heat exchanger (e.g. tube or plate heat exchanger) where the hot solution 20 transfers heat to the cold solution 16, so that the solution 20 is cooled to a suitable temperature for crystallization and the solution 16 is preheated before admission into the evaporator EVAP.

The leaching process in the reactor LSTR and the separation in the separator U-SEP are preferably performed at the same temperature, that is leaching temperature. Very preferably, the inputs UR and LS are also at the leaching temperature and the leaching process is substantially an isothermal one.

It can be appreciated that the processing of the purge solution 122 in the section BCS has the double advantage of controlling the amount of biuret in the leaching process, and obtaining the further valuable product BIU.

The solid urea UG may be fed by a tied-in a urea plant. For example the output of a shaping section of a urea plant may be directly connected to the leaching reactor LSTR.

In can be noted that water is continuously recirculated in the section BCS. Water possibly lost in the loop of the section BCS may be compensated with a suitable make-up, not shown.

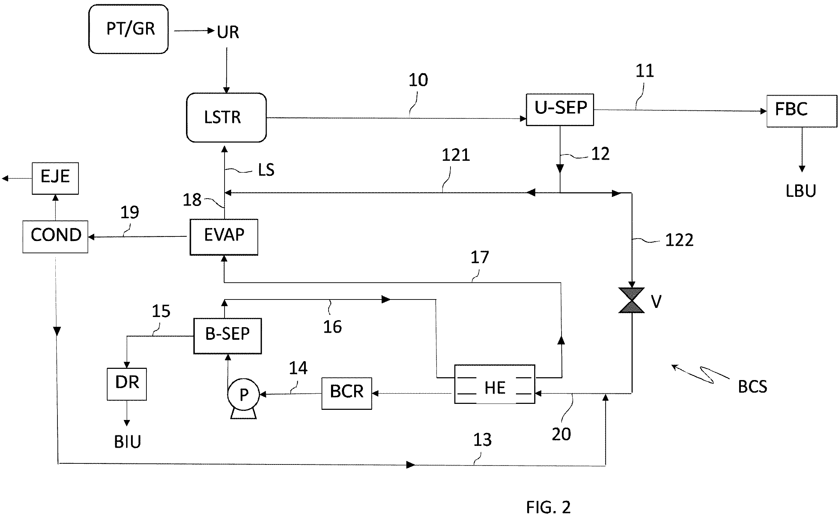

Fig. 2 illustrates an embodiment which differs from Fig. 1 in the final urea product. In Fig. 2, the solid fraction 11 from the separator U-SEP is directed to a fluid bed cooled FBC to produce low-biuret granules of solid urea LBU. Said urea LBU may contain less than 1.0% biuret.

Fig. 2 illustrates also the source of the urea granules UG, which is a prilling tower PT or a urea granulator GR. Said prilling tower or granulator are part of a tied-in urea plant.

It must be noted that Fig. 1 and Fig. 2 are given as examples, and the invention is equally applicable to processes for production of urea for various applications.

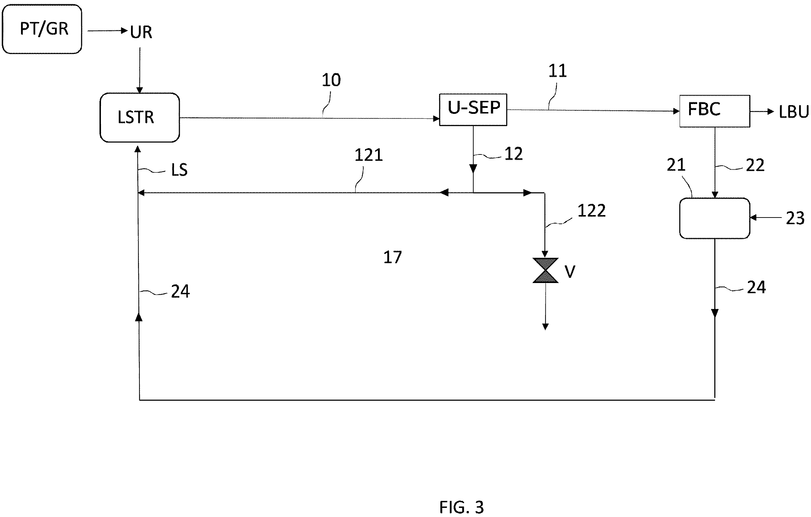

Fig. 3 illustrates a simplified embodiment where the biuret crystallization section BCS is not provided. The purge solution 122 is recycled directly to a tied-in urea plant, for example to the synthesis reactor. The amount of biuret in the reactor LSTR is controlled by injection of a low-biuret urea solution 24 mixed with the main recycle portion 121.

Specifically, in the example of Fig. 3 said low-biuret urea solution 24 is obtained in a dissolver 21 where a portion 22 of the low-biuret solid urea from the cooler FBC is dissolved in a fresh make-up water 23. In other embodiments, solid urea from a different source, such as from storage, may be used in the dissolver 21.

### Example

The following example provides some process conditions with reference to Fig. 1. The leaching process is performed at 60 °C. The purge solution 122 is 5% of the recycle solution 12, the stream 121 including the remainder 95%. After mixing with the water stream 13, the solution 20 contains about 50% of water and is cooled to 15 °C in the heat exchanger (chiller) HE. The slurry 14 after crystallization is at about 5 °C. The re-heating in the hot side of the heat exchanger HE brings the solution 16 to a temperature of 50 °C.

## Claims

1. A process for removing biuret for solid urea (UR), wherein the solid urea is in a granular or powder form, the process including leaching the solid urea with a leaching solution (LS) which is a urea saturated aqueous solution, wherein biuret contained in the solid urea is transferred to said leaching solution during the leaching process, obtaining a purified solid urea, and said purified solid urea is separated from the leaching solution after the leaching process.

2. A process according to claim 1, wherein leaching is performed at a controlled temperature not greater than 80 °C, preferably in the range 20 °C to 80 °C, more preferably in the range 50 °C to 70 °C, even more preferably 55 °C to 65 °C.

3. A process according to any of the previous claims wherein the solid urea is contacted with the leaching solution for a contact time of 15 minutes to 120 minutes.

4. A process according to any of the previous claims wherein the solid urea is contacted with the leaching solution in a stirred tank reactor (LSTR).

5. A process according to any of the previous claims wherein, in the leaching process, the weight ratio between the solid urea to be processed and the leaching solution is between 1.0 and 4.0, preferably between 1.5 and 2.5.

6. A process according to any of the previous claims wherein, after the leaching process, the purified solid urea is separated from the leaching solution by centrifugation and/or by filtering.

7. A process according to any of the previous claims wherein the separation of purified solid urea from the leaching solution is performed at the same temperature or substantially the same temperature as the leaching process.

8. A process according to any of the previous claims wherein the solid urea subject to the leaching process is in the form of spherical granules having an average diameter not greater than 3.5 mm.

9. A process according to any of the previous claims wherein: the leaching solution (12) separated from the purified urea is split into a first portion (121) which is directly recycled to the leaching process, forming a main recycle stream, and a purge solution (122).

10. A process according to claim 9 wherein the concentration of biuret in the leaching solution is controlled:
a) by processing the purge solution (122) to remove biuret contained therein, and joining a so obtained biuret-depleted secondary recycle stream (18) of urea solution with said main recycle stream (121), and/or
b) by adding the main recycle stream with a make-up of fresh aqueous urea solution, said make-up solution containing no biuret or having a content of biuret lower than that of said main recycle stream.

11. A process according to claim 10 wherein the processing of the purge solution (122) according to option a) includes:
the purge solution is diluted with an aqueous stream (13) to reach a concentration of water suitable for crystallization of biuret and is subject to a biuret crystallization process;
a slurry (14) obtained in the biuret crystallization process is separated into a solid phase (15) of biuret-containing crystals and a biuret crystallization liquor (16);
said liquor (16) is concentrated by evaporation to remove water to obtain said secondary recycle stream (18).

12. A process according to claim 11 wherein said evaporation of the biuret crystallization liquor is regulated to obtain a liquid solution saturated with urea.

13. A process according to claim 12 wherein the purge solution, before the crystallization of biuret, is cooled by transferring heat to said crystallization liquor, so that the liquor is pre-heated before the evaporation step.

14. A process according to any of claims 11 to 13 wherein at least part of said aqueous stream (13) added to the purge solution is obtained from water removed from said liquor (16) in the evaporation process.

15. A process according to claim 10 wherein option b) includes that a portion of the purified urea, obtained after the leaching process, is dissolved with water to obtain said make-up solution.

16. A process according to claim 10 wherein option b) includes recycling the purge solution to a tied-in urea urea plant, wherein said purge solution is passed through a urea synthesis reactor of said urea plant.

17. A process according to any of claims 10 to 16 wherein the leaching process is performed in a leaching apparatus and the separation of the solution from the purified urea is performed in a separator, and the amount of said purge solution removed from the leaching solution and/or the amount of make-up solution is controlled to maintain a target concentration of biuret in the leaching solution at the outlet of the leaching apparatus.

18. A process according to claim 17 wherein said target concentration of biuret is below saturation and preferably in the range 15 to 18 wt%.

19. A process according to any of claims 9 to 18 wherein the purge solution is 1% to 20% by weight, preferably 2% to 10% by weight, of the solution effluent from the leaching process.

20. A process according to any of the previous claims wherein a leaching solution, initially containing no biuret, is continuously recycled to the leaching process without removing a purge stream until a target concentration of biuret is reached in the solution effluent from the leaching process.

21. A process for producing low-biuret urea comprising:
an aqueous solution of urea is produced by reacting ammonia and carbon dioxide under urea-forming conditions and subsequent purification of the reaction effluent, preferably according to a urea stripping process;
said aqueous solution of urea is subject to evaporation to remove water and obtain a concentrated urea solution or a urea melt;
said concentrated urea solution or urea melt is processed by prilling or granulation to produce solid urea;
at least a portion of said solid urea is processed to remove biuret in accordance with any of claims 1 to 20.
